# EUROPEAN PATENT SPECIFICATION

(11) **EP 4 200 026 B1**
(45) Date of publication and mention of the grant of the patent: **14.01.2026**
(21) Application number: 22723865.6
(22) Date of filing: 22.04.2022
(51) Int. Cl.: A61Q 11/00, A61K 8/81, A61K 8/90, A61K 8/34, A61K 8/46, A61K 8/36, A61K 8/60, A61K 8/86, A61K 8/368, A61K 8/21, A61K 8/365, A61K 8/73

(54) **SOLID ORAL CARE COMPOSITIONS**
FESTE MUNDPFLEGEZUSAMMENSETZUNGEN
COMPOSITIONS SOLIDES POUR SOIN BUCCODENTAIRE

(30) Priority: 23.04.2021 US 202163178914 P
(43) Date of publication of application: 28.06.2023
(73) Proprietor: Colgate-Palmolive Company, New York, NY 10022 (US)
(72) Inventor: JIMENEZ, Erica, Hamilton, New Jersey 08620 (US); BERRY, Jamal, Jackson, New Jersey 08527 (US); POTNIS, Shashank, Princeton 08540 (US)
(74) Representative: Sonnenhauser, Thomas Martin
(86) International application number: PCT/US2022/026039
(87) International publication number: WO 2022/226369

(56) References cited:
- KR-A- 20040 095 512
- US-A- 6 096 293
- US-A1- 2012 244 203
- US-A1- 2012 263 659
- US-A1- 2019 358 157
- US-A1- 2020 085 864
- US-B2- 8 277 824

## Description

### FIELD OF THE INVENTION

This invention relates to solid oral care compositions, e.g., dissolvable mouthwash compositions in the form of a tablet. In certain aspects this disclosure is directed said solid oral care compositions for use in medical methods and related kits.

### BACKGROUND

Regular use of mouthwashes can reduce the plaque growth in your mouth, decrease your chances of developing gum disease, and prevent tooth decay. While not a replacement for daily brushing, mouthwash offers the benefit of reaching areas not easily accessed by a toothbrush and can provide a deep clean in the mouth. Targeted application of mouthwash not only ensures a healthy oral environment, but also enhances the cosmetic appearance of the user's teeth and limits halitosis. Mouthwash is thus an increasingly important in people's daily oral care routines.

However, since mouthwash is a liquid form, the package can be bulky and inconvenient to carry, and is often inconvenient or restricted to carry when traveling by airplane. Mouthwash containers are also typically sold in and dispensed from large plastic containers, which if not properly recycled, are detrimental to the environment. While these mouthwashes have been traditionally used, there is a market need for products that can be used on the go, require less packaging, can be stored long term and require less water. However, the types of oral care products which address these needs are limited. Tablets are available, but some of these products may have issues with characteristics such as friability, and may break or fall apart during shipment or at some time prior to use by the consumer. Some solid oral care compositions are disclosed in US 2019/358157 A1, KR 2004 0095512 A, US 2020/085864 A1, US 2012/263659 A1, US 6 096 293 A, US 8 277 824 B2 and US 2012/2442013 A1.

Sugar alcohols are commonly used as sweeteners in pharmaceutical and food compositions. However, sugar alcohols such as mannitol are known to cause stickiness during compression. During production of tablets the powder will stick to the upper and lower punch. This leads to many problems including picking which results in imperfections on the face of the tablet. These sugar alcohols have strong adhesive properties towards metal through molecular attraction and are thus also responsible for causing issues in formulation.

Accordingly, there is a need for an oral care product that can possibly be an alternative to the mouthwashes currently on the market. There is also a need to create a stabilized solid oral care composition that can be efficiently manufactured in light of the difficulties associated with sugar alcohols.

### BRIEF SUMMARY

The invention is directed to a solid oral care composition (e.g., a tablet) comprising a sugar alcohol and a lubricant system, wherein the lubricant system comprises a poloxamer (e.g., poloxamer 407) present in an amount as defined in the claims sufficient to maintain stability of the composition for a period of3-30 days (e.g., 3-14 days or 6 days) when dissolved in water, according to claim 1.

The lubricant system comprises a poloxamer (e.g., poloxamer 407), an anionic surfactant, e.g. sodium lauryl sulfate, and a stearate, e.g. magnesium stearate.

In a further aspect, the present invention refers to the solid oral care composition for use in a method for controlling a bacterial population in the oral cavity as defined in the claims, the method comprising dissolving a solid oral care composition in water to form an aqueous solution, and storing the aqueous solution in a container for at least 3 days.

In another aspect, the present disclosure provides a kit comprising (i) a solid oral care composition of the invention; and (ii) a container for holding a liquid for a prolonged period of time

### DETAILED DESCRIPTION

As used throughout, ranges are used as shorthand for describing each and every value that is within the range.

Unless otherwise specified, all percentages and amounts expressed herein and elsewhere in the specification should be understood to refer to percentages by weight. The amounts given are based on the active weight of the material.

Open terms such as "include," "including," "contain," "containing" and the like mean "comprising." In this description, unless otherwise stated, the use of the singular also includes the plural.

As used herein, an "oral care composition" refers to a composition for which the intended use includes oral care, oral hygiene, and/or oral appearance, or for which the intended method of use comprises administration to the oral cavity, and refers to compositions that are palatable and safe for topical administration to the oral cavity, and for providing a benefit to the teeth and/or oral cavity. The term "oral care composition" thus specifically excludes compositions which are highly toxic, unpalatable, or otherwise unsuitable for administration to the oral cavity. In some embodiments, an oral care composition is not intentionally swallowed, but is rather retained in the oral cavity for a time sufficient to affect the intended utility. The oral care compositions as disclosed herein may be used in nonhuman mammals such as companion animals (e.g., dogs and cats), as well as by humans. In some embodiments, the oral care compositions as disclosed herein are used by humans. Solid oral care compositions include, for example, powder (e.g., a free-flowing granulation), tablet, caplet (type of tablet), granule, pellet, wafer, film and bead.

As used herein, "effective amount" refers to an amount of a compound or composition sufficient to induce a positive benefit, a functional benefit to the oral care composition (e.g., a formulation benefit to the composition), an oral health benefit, and/or an amount low enough to avoid serious side effects, i.e., to provide a reasonable benefit to risk ratio, within the sound judgment of a skilled artisan.

As used here, "unit-dose" refers to an amount of the oral care composition to be administered to a patient or consumer in a single use. The unit-dose oral care composition can be a unit-dose powder (e.g., a free-flowing granulation), unit-dose tablet, unit-dose caplet (type of tablet), unit-dose granule, unit-dose pellet, unit-dose wafer, unit-dose film and unit-dose bead or any other suitable unit-dose oral care composition capable of being retained in the oral cavity for a time sufficient to contact some or all of the dental surfaces and/or oral tissues for purposes of oral health.

In certain aspects, the solid oral care compositions may be stored in an air tight, moisture-proof package, e.g., sachets, sealed metal foil pouches, blister packs, and desiccant capped tubes. Useful packaging materials include polymeric packaging (e.g., polyethylene and polypropylene), metal foil packaging (e.g., aluminum), and combinations thereof.

The solid oral care compositions of the present disclosure preferably contain no water or have a low water content. As used herein, the term "low water content" means the total concentration of water, including any free water and all water contained in any ingredients. In various embodiments of the composition, the amount of water is in an amount of less than 4% by weight, or less than 3% by weight, or less than 2% by weight, or less than 1% by weight, or less than 0.5% by weight, or less than 0.1%, or about 0.0001% to about 4% by weight, or about 0.0001% to about 0.5% by weight or about 0.0001% to about 0.1% by weight.

The solid oral care compositions of the present disclosure can be in a variety of forms including, e.g., powder (e.g., a free-flowing granulation), tablet, caplet (type of tablet), granule, pellet, wafer, film and bead.

The solid oral care compositions comprises a sugar alcohol. In various aspects, the term sugar alcohol refers to polyhydroxy alcohols that include cyclic or acyclic polyols. Acyclic sugar alcohols have the general formula CnHn+2(OH)n. Preferred sugar alcohols are those containing four to six carbon atoms (i.e., n is 4 to 6), especially five or six carbon atoms (n is 5 or 6). In various embodiments, the sugar alcohol is selected from the group consisting of mannitol, sorbitol, erythritol, xylitol, lactitol, maltitol, isomalt and combinations thereof. The sugar alcohol may be present in an amount between 1-20 wt. %, based on the total weight of the composition, for example between about 3-15 wt. %, based on the total weight of the composition, for example between about 5-10 wt. %, based on the total weight of the composition.

In certain aspects, the solid oral care compositions of the present disclosure contain a buffering agent. Examples of buffering agents include anhydrous carbonates such as sodium carbonate, sesquicarbonates, bicarbonates such as sodium bicarbonate, silicates, bisulfates, phosphates such as monopotassium phosphate and dipotassium phosphate, citrates, pyrophosphates (sodium and potassium salts) and combinations thereof.

In certain aspects, the solid oral care compositions further comprises a polymeric binder which adds bulk to the compositions and assists in holding the components of the composition together when in the form of a tablet. Examples of suitable polymeric binders include, e.g., starches, natural gums, (e.g., xanthan gum), cellulose gums, microcrystalline cellulose, maltodextrins, methylcellulose, cellulose ethers, sodium carboxymethylcellulose, ethylcellulose, gelatin, polyethylene glycol, polypropylene glycol, co-polymers of polyethylene glycol and polypropylene glycol (e.g., poloxamers), pectins, alginates, polyacrylamides, polyvinylpyrrolidone, polyvinyloxozolidone, polyvinyl alcohols and mixtures thereof.

In certain aspects, the solid oral care compositions of the invention can include an acid buffering agent. For example, these acids can include citric acid, ascorbic acid, malic acid, adipic acid, tartaric acid, fumaric, succinic acid, sodium acid pyrophosphate, lactic acid, hexamic acid, and acid salts and acid anhydrides thereof, and mixtures thereof Examples of useful acid anhydrides include citraconic anhydride, glucono-D-lactone, and succinic anhydride. Examples of useful acid salts include potassium bitartrate, acid citrate salts, sodium dihydrogen phosphate, disodium dihydrogen phosphate, sodium acid sulfite, and combinations thereof.

In certain aspects, the solid oral care compositions of the invention can include a carbonate base. Examples of suitable carbonate bases include sodium bicarbonate, sodium carbonate, sodium sesquicarbonate, potassium carbonate, potassium bicarbonate, calcium carbonate, magnesium carbonate, magnesium oxide, sodium glycine carbonate, L-lysine carbonate, arginine carbonate, zinc carbonate, and mixtures thereof. In certain aspects, the base is present in the composition in an amount of 5% by weight to 60% by weight, about 7% by weight to 50% by weight, or about 10% by weight to about 40% by weight, or about 20% by weight to about 30% by weight.

The solid oral care compositions of the invention as defined in the claims comprise a lubricant system comprising lubricants as defined in the claims. Various lubricants are suitable for use in the composition including water dispersible, water soluble, water insoluble lubricants and combinations thereof. Examples of useful water-soluble lubricants include sodium benzoate, polyethylene glycol, L-leucine, adipic acid, and combinations thereof. The composition includes stearates (e.g., magnesium stearate, calcium stearate and zinc stearate), and the composition can also include other water insoluble lubricants including, e.g., oils (e.g., mineral oil, hydrogenated and partially hydrogenated vegetable oils, and cotton seed oil) and combinations thereof. Other water insoluble lubricants include, e.g., animal fats, polyoxyethylene monostearate, talc, and combinations thereof When the composition is in the form of a tablet, the composition preferably includes a sufficient amount of lubricant to enable the composition to be formed into tablets and released from a high speed tableting press in the form of a tablet.

In certain aspects, the solid oral care compositions described herein can further include additional ingredients, e.g., flavor agents; fillers; surfactants; and dyes and pigments; and sweeteners.

The solid oral care compositions comprise anionic surfactants, for example, water-soluble salts of higher fatty acid monoglyceride monosulfates, such as the sodium salt of the monosulfated monoglyceride of hydrogenated coconut oil fatty acids such as sodium cocoyl glutamate, sodium N- methyl N-cocoyl taurate, sodium cocomo-glyceride sulfate; higher alkyl sulfates, such as sodium lauryl sulfate; higher alkyl-ether sulfates, e.g., of formula CH₃(CH₂)ₘCH2(OCH₂CH₂)ₙOSO₃X, wherein m is 6-16, e.g., 10, n is 1-6, e.g., 2, 3 or 4, and X is Na or , for example sodium laureth-2 sulfate (CH₃(CH₂)₁₀CH₂(OCH₂CH₂)₂OSO₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2-ethyl laurate potassium sulfoacetamide) and sodium lauryl sarcosinate. By "higher alkyl" is meant, e.g., C6-3o alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5% or 2.5%.

In certain aspects, solid oral care compositions can further comprise one or more cationic surfactants. Cationic surfactants that are useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition of the invention comprises a nonionic surfactant selected from poloxamers (e.g., poloxamer 407), polysorbates (e.g., polysorbate 20), polyoxyl hydrogenated castor oils (e.g., polyoxyl 40 hydrogenated castor oil), and mixtures thereof.

The surfactant or mixtures of compatible surfactants can be present in the compositions of the present invention in 0.1% to 5%, in another embodiment 0.3% to 3% and in another embodiment 0.5% to 2.5% by weight of the total composition.

In certain aspects, the solid oral care compositions described herein can comprise one or more fillers. For example, the filler can be one or more selected from: crystalline cellulose, ethylcellulose, dextrin, various kinds of cyclodextrin (α-cyclodextrin, β-cyclodextrin and γ-cyclodextrin), sodium sulfate, as well as derivatives thereof and pullulan.

In certain aspects, solid oral care compositions can further comprise one or more flavoring agents. Useful flavor agents include natural and synthetic flavoring sources including, e.g., volatile oils, synthetic flavor oils, flavoring aromatics, oils, liquids, oleoresins and extracts derived from plants, leaves, flowers, fruits, stems and combinations thereof. Suitable flavor agents include, e.g., citric oils, e.g., lemon, orange, grape, lime and grapefruit, fruit essences including, e.g., apple, pear, peach, grape, strawberry, raspberry, cherry, plum, pineapple, apricot, and other fruit flavors. Other useful flavor agents include, e.g., aldehydes and esters (e.g., benzaldehyde (cherry, almond)), citral, i.e., alpha-citral (lemon, lime), neral, i.e., beta-citral (lemon, lime), decanal (orange, lemon), aldehyde C-8 (citrus fruits), aldehyde C-9 (citrus fruits), aldehyde C-12 (citrus fruits), tolyl aldehyde (cherry, almond), 2,6-dimethyloctanal (green fruit), 2-dodedenal (citrus, mandarin) and mixtures thereof.

In certain aspects, the solid oral care compositions described herein can comprises one or more dyes, lakes. Useful lakes include dyes absorbed on aluminum hydroxide and other suitable carriers.

In certain aspects, solid oral care compositions described herein can comprise one or more sweetener, e.g., selected from: stevia, sugars such as sucrose, glucose, invert sugar, fructose, ribose, tagalose, sucralose, malitol, erythritol, xylitol, and mixtures thereof, saccharin and its various salts (e.g., sodium and calcium salt of saccharin), cyclamic acid and its various salts, dipeptide sweeteners (e.g., aspartame), acesulfame potassium, dihydrochalcone, glycyrrhizin, and combinations thereof.

In certain aspects, the solid oral care compositions of the invention can comprise one or more polymers, such as polyethylene glycols, polyvinylmethyl ether maleic acid copolymers, polysaccharides (e.g., cellulose derivatives, for example carboxymethyl cellulose, or polysaccharide gums, for example xanthan gum, acacia gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include about 1:4 to about 4:1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139(M.W. 500,000), AN 119 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

In certain aspects, the solid oral care compositions may further comprise one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al..

Representative fluoride ion sources include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof.

In certain embodiments, the oral care composition of the invention may comprise a source of fluoride ions or fluorine-providing ingredient in amounts sufficient to supply about 25 ppm to 25,000 ppm of fluoride ions, generally at least about 500 ppm, e.g., about 500 to about 2000 ppm, e.g., about 1000 to about 1600 ppm, e.g., about 1450 ppm. The appropriate level of fluoride will depend on the particular application.

Fluoride ion sources may be added to the compositions of the invention at a level of about 0.01 wt. % to about 10 wt. % in one embodiment or about 0.03 wt. % to about 5 wt. %, and in another embodiment about 0.1 wt. % to about 1 wt. % by weight of the composition in another embodiment. Weights of fluoride salts to provide the appropriate level of fluoride ion will obviously vary based on the weight of the counter ion in the salt. Preparing oral care compositions, it is sometimes necessary to add some thickening material to provide a desirable consistency or to stabilize or enhance the performance of the formulation. In certain aspects, the solid oral care compositions can comprise one or more thickening agents selected from: carboxyvinyl polymers, carrageenan, and water-soluble salts of cellulose ethers such as sodium carboxymethyl cellulose and sodium carboxymethyl hydroxyethyl cellulose. Natural gums such as karaya, gum Arabic (i.e., acacia gum), and gum tragacanth can also be incorporated. Colloidal magnesium aluminum silicate or finely divided silica can be used as component of the thickening composition to further improve the composition's texture. In certain embodiments, thickening agents in an amount of about 0.5% to about 10.0% by weight of the total composition are used.

In various embodiments of the disclosure, the oral care compositions disclosed herein may comprise at least one zinc salt. In certain embodiments, the at least one zinc salt is a water-soluble zinc salt, such as zinc chloride or zinc lactate. Other exemplary zinc salts that may be mentioned include zinc oxide, zinc sulfate, zinc citrate, zinc gluconate, zinc malate, zinc tartrate, zinc carbonate, and zinc phosphate. In some embodiments, the oral care compositions disclosed herein may comprise from about 0.01 to about 15 weight % of a zinc salt, such as about 5 to about 15 weight % of a zinc salt, about 10 to about 15 weight % of a zinc salt, about 10 to about 13 weight % of a zinc salt, about 11 weight % of a zinc salt, e.g. about 11.2 weight % of a zinc salt.

It is understood that while general attributes of each of the above categories of materials may differ, there may be some common attributes and any given material may serve multiple purposes within two or more of such categories of materials. All of the ingredients in the compositions may have functions in addition to their primary function, and may contribute to the overall properties of the composition, including its stability, efficacy, consistency, mouthfeel, taste, odor and so forth. For example, a binder may also function as a disintegrating agent and vice versa.

In certain aspects, the solid compositions of the present disclosure can be made via techniques known in the art. Documents which disclose techniques which may be used to prepare the solid compositions of the present disclosure are US patents 4,886,669; 6,106,861; 6,596,311; 6,743,443; 6,811,793; 7,501,409; 7,815,897; 8,377,995; and US patent application 2005/0169986. In some embodiments, the ingredients and optional components can be kneaded with an organic solvent, filled in a mold and subjected to a compression-molding. The organic solvent can be an alcohol such as methanol, ethanol, propanol, isopropanol. The kneading and granulating operations carried out by adding such auxiliary agents for making the preparation and by adding such a solvent may be conducted using the conventionally used apparatus. For example, a fluidized bed granulator, a tumbling granulator, an extrusion granulator or a spray-drying drier may be used. The solid compositions may also be prepared via freeze drying.

In some embodiments, the solid compositions of the present disclosure, for example tablets, can be prepared by a direct compression process, wherein the constituent components of the tablet are blended together and then the mixture is compressed into tablets.

In certain aspects, granules can be prepared by any one of known methods for preparing granules such as dry granulation, layering granulation, impregnated-granulation, etc. For dry granulation, a mixture of ingredients with optional additive(s) is subjected to granulation with a roller compactor, a roll granulator, etc.

For layering granulation, a mixture similar to the above is added to a rolling inactive carriers while spraying a binder solution with a centrifugal fluidized bed granulator or the like to make the mixture adhere to the carries. Examples of the inactive carrier that used in this method include crystals of sugars or inorganic salts such as crystalline lactose, crystalline cellulose, crystalline sodium chloride, etc., and spherical granules such as spherical granules of crystalline cellulose (brand name: Avicel SP, Asahi Kasei Corporation), spherical granules of crystalline cellulose and lactose (brand name: Nonpareil-NP-5 and NP-7, Freund Co., Ltd.), spherical granules of purified white sugar (brand name: Nonpareil-103, Freund Co., Ltd.), spherical granules of lactose and α starch, etc.

For impregnating granulation, a solution containing potassium peroxymonosulfate and other ingredients at an appropriate concentration is mixed with porous carriers thereby a sufficient amount of solution is made to retain in the cavities of the carrier, which is followed by drying to remove the solvent. Examples of the porous carrier that can be used include magnesium aluminometasilicate (brand name: Neusiline, Fuji Chemical Industry Co., Ltd.), calcium silicate (Florite, Eisai Co., Ltd.), etc. Examples of the solvent include ethanol, methanol, or the like.

In certain aspects, the solid compositions of the present disclosure can include polyvinylpyrrolidone (PVP). The PVP can be present as a PVP polymer such as povidone, or as a copolymer of polyvinylpyrrolidone and another constituent such as vinyl acetate. Examples of such copolymer include the Plasdone^{™} polymers, e.g. Plasdone^{™} S630, available from Ashland Chemical Co.

The invention is directed to a solid oral care composition (e.g., a tablet) comprising:
a sugar alcohol;
a lubricant system comprising:
   a poloxamer being present in an amount sufficient to maintain stability of the composition for a period of 3-30 days when dissolved in water wherein the amount of the poloxamer is from 0.1 to 0.5 wt.%, relative to the total weight of the solid oral care composition,
   an anionic surfactant, and
   a stearate, and
      a preservative comprising sodium benzoate and potassium sorbate; and wherein the sodium benzoate is present an amount of from 15 to 25 wt.%, relative to the total weight of the composition, and the potassium sorbate is present in an amount of 1 to 8% by weight relative to the total weight of the solid oral care composition.

The invention is as defined in the claims and contemplates any of the following aspects (unless otherwise indicated, values are given as percentage of the overall weight of the composition):
The solid oral care composition may further comprise polyvinylpyrrolidone (PVP) or a copolymer thereof.

The composition may comprise a copolymer of polyvinylpyrrolidone.

The polyvinylpyrrolidone may be a copolymer of polyvinylpyrrolidone and vinyl acetate.

The composition may comprise PVP or a copolymer thereof and the poloxamer in a weight ratio of 9:1 to 1:1 (PVP or copolymer thereof : poloxamer), e.g., 6:1 to 2:1 (PVP or copolymer thereof : poloxamer), e.g., 4.5:1 (PVP or copolymer thereof : poloxamer), or 25:1 to 1:1, e.g., 24:1 to 15:1 (PVP or copolymer thereof : poloxamer), or 22:1 to 18:1 (PVP or copolymer thereof : poloxamer), e.g., 21:1 (PVP or copolymer thereof : poloxamer), e.g. 20:1 (PVP or copolymer thereof : poloxamer). wherein the weight is relative to the total weight of the composition.

The composition may comprise PVP or a copolymer thereof and poloxamer in a weight ratio of 4.5:1 (PVP : poloxamer), or a weight ratio of 21:1 (PVP : poloxamer), wherein the weight is relative to the total weight of the composition.

The amount of PVP or copolymer thereof may be from 1% - 15% by weight of the total composition.

The amount of PVP or copolymer thereof may be from 2% - 6% by wt. of the total composition (e.g., 4 - 5 %).

The amount of PVP or copolymer thereof may be 4% to 4.5% by wt. of the total composition. The PVP may be a cross-linked polyvinylpyrrolidone (e.g., crospovidones).

The PVP may be a copolymer of polyvinylpyrrolidone and vinyl acetate (e.g., Plasdone^{™} S630).

The poloxamer may be poloxamer 407.

The lubricant system further comprises a stearate, e.g. magnesium stearate, calcium stearate, zinc stearate or a combination thereof.

The amount of stearate may be from 0.1% - 0.5% by weight of the total composition, e.g. about 0.3% by weight of the total composition.

The stearate may be magnesium stearate.

The sugar alcohol may be selected from the group consisting of mannitol, sorbitol, erythritol, xylitol, lactitol, maltitol, isomalt and combinations thereof.

The sugar alcohol is selected from mannitol and sorbitol.

The sugar alcohol may comprise mannitol and sorbitol, in a weight ratio of mannitol : sorbitol of 0.8 : 1 to 1.2 : 1, for example about 1 : 1.

The sugar alcohol comprises or consists of mannitol.

The sugar alcohol may be present in an amount between 1-20 wt. %, based on the total weight of the composition, for example between about 3-17 wt. %, based on the total weight of the composition, for example between about 5-15 wt. %, based on the total weight of the composition, for example about 11 wt. % or about 14 wt. %, based on the total weight of the composition; or the sugar alcohol is present in an amount between 10-30 wt. %, based on the total weight of the composition, for example between about 15-25 wt. %, based on the total weight of the composition, for example between about 18-22 wt. %, based on the total weight of the composition, for example about 20 wt. % based on the total weight of the composition,

The preservative may be present in an amount sufficient to maintain antimicrobial efficacy following dissolution of the solid oral care composition for a period of at least 4 days, at least 5 days, at least 6 days, at least 7 days, or at least 14 days.

The preservative is present in an amount to maintain sufficient to maintain antimicrobial efficacy following dissolution of the solid oral care composition for a period of 3 days, 4 days, 5 days, 6 days, or 7 days.

The compositions comprise a preservative, wherein the preservative is present in an amount sufficient to maintain antimicrobial efficacy following dissolution of the solid oral care composition for a period of 3 to 30 days, wherein the preservative comprises sodium benzoate and potassium sorbate; and wherein the sodium benzoate is present an amount of from 15 to 25 wt.%, relative to the total weight of the composition, and the potassium sorbate is present in an amount of 1 to 8% by weight relative to the total weight of the solid oral care composition, as defined in the claims .

The compositions comprise sodium benzoate in an amount of 15-25% by weight (e.g., 15% or 20%), based on the total weight of the composition, as defined in the claims.

The compositions comprise potassium sorbate in an amount of 1-8% by weight (e.g., 2% or 6%), based on the total weight of the composition, as defined in the claims.

The compositions comprise sodium benzoate in an amount of 15-25% by weight (e.g., 20%), and potassium sorbate in an amount of 1-5% by weight (e.g., 2%), or 1-8% by weight (e.g., 6%), based on the total weight of the composition.

The composition may be in the form of a tablet, powder or granule.

The composition may be in the form of a tablet.

The composition may be a single unit-dose oral care composition.

The composition may contain no water or water in an amount of less than 4%, or less than 3%, or less than 2%, or less than 1%, or less than 0.5%, or from 0.0001% to 4%, or 0.0001% to 0.5% or 0.0001% to 0.1%, or 0.001% to 4%, by weight.

The composition may further comprise a polymeric binder which adds bulk to the compositions and assists in holding the components of the composition together when in the form of a tablet. The polymer binder may be selected from starches, natural gums (e.g., xanthan gum or acacia gum), cellulose gums, microcrystalline cellulose, maltodextrins, methylcellulose, cellulose ethers, sodium carboxymethylcellulose, ethylcellulose, gelatin, polyethylene glycol, cross-linked polyvinylpyrrolidone, pectins, alginates, polyacrylamides, polyvinyloxazolidone, polyvinyl alcohols and mixtures thereof in an amount of about 1-5 wt. %, based on the total weight of the composition, e.g., about 2 wt. % based on the total weight of the composition.

The composition may further comprise acacia gum.

The polymer binder may comprise or consist of hydroxypropylcellulose.

The binder may further comprises a non-polymeric binder.

The non-polymeric binder may be xanthan gum.

The composition may contain no binder.

The composition may contain no gum (e.g., acacia gum).

The composition may further comprise a buffering agent selected from a silicate, a bisulfate, a citrate, a phosphate (e.g., monopotassium phosphate), dipotassium phosphate, and combinations thereof.

The buffering agent may be in an amount of from 10.0 - 40% or about 20 % - 30% by weight, based on the total weight of the composition of the total composition.

The buffering agent may be citric acid.

The composition may comprise an acid buffering agent selected from: citric acid, ascorbic acid, malic acid, adipic acid, tartaric acid, fumaric acid, succinic acid, sodium acid pyrophosophate, lactic acid, hexamic acid, citraconic anhydride, glucono-D-lactone, succinic anhydride, potassium bitartrate, acid citrate salts, sodium dihydrogen phosphate, disodium dihydrogen phosphate, and sodium acid sulfite.

The composition may further comprise a carbonate base selected from anhydrous carbonate (e.g., sodium carbonate), a sesquicarbonate, a bicarbonate (e.g., sodium bicarbonate) in an amount of about 10 - 40% by weight (e.g., about 20-30% or about 15-20% by weight), based on the total weight of the composition.

The carbonate base may be selected from sodium bicarbonate, sodium carbonate, sodium sesquicarbonate, potassium carbonate, potassium bicarbonate, calcium carbonate, magnesium carbonate, magnesium oxide, sodium glycine carbonate, L-lysine carbonate, arginine carbonate, and zinc carbonate.

The carbonate base may be sodium bicarbonate.

The composition may additionally comprise one or more flavor agents, one or more fillers, one or more surfactants, one or more dyes or lakes, or any combination of two or more thereof.

The compositions comprise a lubricant system wherein the lubricant system further comprises an anionic surfactant (e.g., from 1% - 5% by wt.) (e.g., about 2.5% by wt.), as defined in the claims. The lubricant system may further comprise an anionic or non-ionic surfactant selected from: sodium cocoyl glutamate, sodium lauryl sulfate, sorbitan fatty acid ester, polyoxyethylene (20) sorbitan monooleate (Polysorbate 80 or Tween 80), polyethylene glycol fatty acid ester, polyoxyethylene sorbitan fatty acid ester, polyoxyethylene alkyl ether, polyoxyethylene polyoxypropylene alkyl ether, polyoxyethylene polyoxypropylene block copolymer, polyoxyethylene alkyl phenyl ether, polyoxyethylene castor oil, polyoxyethylene hydrogenated castor oil, polyoxyethylene sorbitol fatty acid ester, polyoxyethylene glycerol fatty acid ester, and combinations thereof (e.g., from 1% - 5% by wt.) (e.g., about 2.5% by wt.); for example sodium lauryl sulfate from 1% - 5% by wt., e.g., about 2.5% by wt.

The composition may comprise a nonionic surfactant.

The solid oral care composition may comprise a nonionic surfactant selected from polysorbates (e.g., polysorbate 20), polyoxyl hydrogenated castor oils (e.g., polyoxyl 40 hydrogenated castor oil), and mixtures thereof (e.g., from 1% - 5% by wt.) (e.g., about 2% by wt.).

The solid oral care composition may comprise a fluoride source.

The fluoride source may be selected from stannous fluoride, sodium fluoride, potassium fluoride, sodium monofluorophosphate, sodium fluorosilicate, ammonium fluorosilicate, amine fluoride, ammonium fluoride, and combinations thereof.

The fluoride source may be sodium monofluorophosphate.

The fluoride source may be in an amount from 0.5% - 2% (e.g., about 0.8%) by weight, based on the total weight of the composition.

The solid oral care composition may be a tablet that comprises:
about 5% - 10% of a sugar alcohol (e.g., mannitol);
poloxamer 407 present in an amount from 0.1% - 0.5% relative to the total composition, as defined in the claims;
up to 25% total of said preservative source comprising sodium benzoate (e.g., about 20% by weight) and potassium sorbate (e.g., about 2% by weight), as defined in the claims; and
From 0.5% - 2% by wt., (e.g., about 0.8%) of sodium fluoride,
wherein all weights are relative to the total weight of the composition.

The lubricant system may comprise:
poloxamer 407 (e.g., about 0.2%) relative to the total composition;
about 1-5% of an anionic surfactant, e.g. 1.5% sodium lauryl sulfate; and
about 0.1% - 0.5% by weight of a stearate, e.g. about 0.3% magnesium stearate.

The solid oral care composition may be a tablet that comprises:
a lubricant system comprising:
poloxamer 407 (e.g., about 0.2%) relative to the total composition;
about 1-5% of an anionic surfactant, e.g. 1.5% sodium lauryl sulfate; and
about 0.1% - 0.5% by weight of a stearate, e.g. about 0.3% magnesium stearate.

The composition may further comprise:
about 18% - 22% of a sugar alcohol (e.g., mannitol and sorbitol in a weight ratio of about 1:1); and
up to 25% total of a preservative source comprising sodium benzoate (e.g., about 20% by weight) and potassium sorbate (e.g., about 2% by weight).

The composition may further comprise 2% - 6% by wt. (e.g., 4 - 5%) PVP or a copolymer thereof, for example a copolymer of PVP and vinyl acetate.

The composition may further comprise:
about 1% - 5% by weight, e.g., about 2% by weight of a binder, e.g.
hydroxypropyl cellulose;
about 20 % - 30% by weight of a buffering agent, e.g. citric acid; and
about 15-20% of a carbonate base, for example sodium bicarbonate.

The solid oral care composition may comprise a fluoride source, wherein the fluoride source is provided in an amount effective to supply from 25 ppm to 25,000 ppm of fluoride ions to the oral cavity (e.g., from 500 to about 2000 ppm) (e.g., 1000 to 1600 ppm) (e.g., 1000ppm) (e.g., about 1450 ppm).

The solid oral care composition may be selected from the group consisting of: a powder (e.g., a free-flowing granulation), tablet, granule, pellet, wafer, film and bead.

The solid oral care composition may be a tablet or granule.

The solid oral care composition may be a tablet.

The composition may be stable for a period of 3-14 days (e.g., 6 days) when dissolved in water.

In a further aspect, the present disclosure provides a solid oral care composition according to the claims for use in a method of controlling a bacterial population in the oral cavity [Method 1], the method comprising dissolving a solid oral care composition in water to form an aqueous solution, and storing the aqueous solution in a container for at least 3 days.

The invention is as defined in the claims and contemplates a solid oral care composition for use in any of the following methods (unless otherwise indicated, values are given as percentage of the overall weight of the composition):
The solid oral care composition may be dissolved in 40mL water per gram of the solid oral care composition.

The method may further comprise the step of using the aqueous solution (i.e., rinsing and/or gargling, and expectorating the solution) daily for a period of at least 3 days.

The method may comprise the step of using the aqueous solution for a period of 5 days, 6 days, 7 days, 8 days, 9 days, 10 days, 11 days, 12 days, 13 days, or 14 days.

The aqueous solution may be used daily as necessary.
the aqueous solution may be used twice daily.

The aqueous solution may be used once daily.

The solid oral care composition may be dissolved in water at a ratio of 30-50 mL (e.g., 40 mL) water per gram of the solid oral care composition.

In one aspect, the invention is directed to a kit comprising (i) a solid oral care composition according to the claims, wherein the poloxamer (e.g., poloxamer 407) is present in an amount sufficient to maintain stability of the composition for a period of 3-30 days (e.g., 3-14 days or 6 days) when dissolved in water wherein the amount of the poloxamer is from 0.1 to 0.5 wt.%; and (ii) a container for holding a liquid for a prolonged period of time.

The kit is as defined in the claims and contemplates any of the following aspects (unless otherwise indicated, values are given as percentage of the overall weight of the composition):
The solid oral care composition of the kit may be dissolved in water held within the container.

The container may comprise a first set of graduations for filling with water.

The first set of graduations may provide one or more markers which indicate fill lines for a volume of water.

The container may comprise a second set of graduations.

The second set of graduations may provide markers which indicate dosage volumes.

The second set of graduations may comprise markers for up to 2 weeks of doses.

The second set of graduations may comprise markers for up to one week of doses.

The second set of graduations may comprise markers for up to one week of doses, e.g., up to 6 days of doses, e.g., up to 5 days of doses, e.g., up to 4 days of doses, e.g., up to 3 days of doses.

The second set of graduations may indicate 10 mL measurements of liquid.

The container may comprise a lid.

The kit may further comprise a dosing cup.

### EXAMPLES

Exemplary compositions will be illustrated by reference to the following examples, which are included to exemplify, but not to limit the scope of the present invention.

### EXAMPLE 1

Sugar alcohols (mannitol) are known to cause stickiness during compression. These sugar alcohols are highly viscous and are thus also responsible for causing rheological issues in formulation. During production of tablets the powder will stick to the upper and lower punch. This leads to many problems including picking which results in imperfections on the face of the tablet.

Formulations were created using a variety of lubricants in order to attempt to mitigate this effect. Magnesium stearate is widely used as a lubricant for tablets. However, it is not soluble in water, and results in a cloudy solution with particulate settling on the bottom of container. Select water-soluble lubricants were evaluated.

Polyvinylpyrrolidone was shown to provide very good binding properties and remains clear up to a certain percentage. Several levels of PVP were tested, summarized in Table 1 below.

**Table 1**

| **Concentration of Polyvinylpyrrolidone** | **Effect** |
|---|---|
| 1.0% | Sticking to tooling, clear solution |
| 3.0% | Sticking improved but still observed after making several tabs, clear solution |
| 4.5% | Sticking improved but still observed after making several tabs, clear solution |
| 6.0% | No sticking observed even after 20+ tablets, solution appears to be clear |
| 6.5% | No sticking but solution is cloudy residue on glass |

Poloxamer 407 was shown to have good anti-adherent properties and is highly water soluble, however adding too much negatively impacted tablet disintegration time significantly. Several levels of poloxamer 407 were tested, summarized in Table 2 below.

**Table 2**

| **Concentration of Poloxamer 407** | **Effect** |
|---|---|
| 1.0% | sufficient lubrication, 1min 50sec disintegration time (20ml and 120ml) |
| 2.0% | good lubrication some sticking, 3 min disintegration time (20ml and 120ml) |
| 3.0% | good lubrication, 5 min disintegration time (20ml) |
| 6.0% | very good lubrication, waxy tab- sticky to the touch (20ml) |

Further testing showed that in order to avoid formation of a cloudy solution, the ratio of solute to solution is critical. 3.0% by weight or higher greatly increases dissolution time.

The results show overall that a water-soluble lubricant should be used. A combination of 4.5% PVP and 1% Poloxamer is used to provide sufficient anti-adherent properties while ensuring solution remains clear, and maintains the disintegration time under 3 min.

### EXAMPLE 2

Using a single punch press, tablets were formed containing sugar alcohols with the lubricant system discussed in Example 1. After dissolving the tablets with 120 mL water, the final oral rinse has a preservative system robust enough to preserve the product for 7 days. The following formulas were tested under a 7 day antimicrobial preservation efficacy test.

Test formulations are inoculated with mixed batteries of microorganisms of known quantities (bacterial and fungal) and monitored for a period of 7 days. Passing formulations result in a log reduction of bacteria of at least 3.0.

**Table 3**

| **Ingredient** | **Formulat ion 1 (wt. %)*** | **Formulat ion 2 (wt. %) *** | **Formulat ion 3 (wt. %) *** | **Formulat ion 4 (wt. %) *** | **Formulati on 5 (wt. %) *** | **Formulati on 6 (wt. %) *** |
|---|---|---|---|---|---|---|
| Poloxamer 407 | 1 | 1 | 16 | 1 | 1 | 1 |
| Polyvinylpyrroli done | 4.5 | 4 | 4.5 | 4.5 | 4.5 | 4.5 |
| D-Mannitol | 8.452 | 7.992 | 8.452 | 8.452 | 8.452 | 6.252 |
| Sodium Benzoate | 3 | 3 | 20 | 20 | 20 | 20 |
| Potassium Sorbate | 6 | 6 | 2 | 2 | 2 | 6 |
| Benzyl Alcohol | 0 | 1 | 0 | 0 | 0 | 0 |
| Sodium Fluoride | 2 | 2 | 2 | 2 | 2 | 2 |
| Sodium Bicarbonate | 28.5 | 28.5 | 17 | 23.57 | 23.57 | 23.5 |
| Citric Acid | 32.8 | 32.8 | 19.6 | 28.03 | 28.03 | 28 |
| Acacia Gum Powder | 2 | 2 | 2 | 2 | 2 | 2 |
| Sorbitol | 10 | 10 | 7 | 7 | 7 | 5 |
| Flavorants and colorants | 1-10 | 1-10 | 1-10 | 1-10 | 1-10 | 1-10 |
| Result: | FAIL | FAIL | FAIL | PASS | PASS | PASS |

| | | | | | | |
|---|---|---|---|---|---|---|
| * not claimed | | | | | | |

Formulation 4 initially showed promising results, but the high level of poloxamer 407 and reduction in effervescence (citric acid + Sodium Bicarbonate) resulted in an unacceptable increase in disintegration time. Poloxamer407 was subsequently decreased to 1% and the effervescence increased. Formulations 5 and 6 with 20% sodium benzoate and 2% potassium sorbate passed Day 7 APET. Formulation 7 with 20% sodium benzoate and 6% potassium sorbate also passed Day 7 APET.

Further formulations were developed, which contained reduced amounts of fluoride. The formulations and results are outlined below in Table 4.

**Table 4**

| **Ingredient** | **Formulation 7 (wt. %) *** | **Formulation 8 (wt. %) *** | **Formulation 9 (wt. %) *** |
|---|---|---|---|
| Poloxamer 407 | 1 | 1 | 1 |
| Polyvinylpyrrolidone | 4.5 | 4.5 | 4.5 |
| D-Mannitol | 8.752 | 8.752 | 7.452 |
| Sodium Benzoate | 20 | 20 | 20 |
| Potassium Sorbate | 2 | 2 | 6 |
| Sodium Bicarbonate | 24.4 | 24.4 | 23.5 |
| Citric Acid | 28.1 | 28.1 | 28 |
| DC Sorbitol | 6.7 | 6.7 | 5 |
| Acacia Gum Powder | 2 | 2 | 2 |
| Sodium Fluoride | 0.8 | 0.8 | 0.8 |
| Flavorants and colorants | 1-3 | 1-3 | 1-3 |
| Total | 100 | 100 | 100 |
| Micro Result: | PASS | PASS | PASS |

| | | | |
|---|---|---|---|
| * not claimed | | | |

Further formulations were developed, which contained no fluoride. These formulations are summarized below in Table 5.

**Table 5**

| **Ingredient** | **Formulation 10 (wt. %) *** | **Formulation 11 (wt. %) *** | **Formulation 12 (wt. %)*** |
|---|---|---|---|
| Poloxamer 407 | 1 | 1 | 0.2 |
| Sodium Benzoate | 20 | 20 | 20 |
| Potassium Sorbate | 6 | 6 | 6 |
| Sodium Bicarbonate | 23.5 | 23.5 | 27.3 |
| Citric Acid | 24.3 | 24.3 | 29.2 |
| Sucralose | 0.9 | 0.9 | 0.9 |
| Sodium Saccharin | 0.3 | 0.3 | 0.3 |
| D-Mannitol | 10.4 | 11 | 11 |
| Polyvinylpyrrolidone | 0 | 0 | 0 |
| DC Sorbitol | 7 | 8 | 0 |
| Acacia Gum Powder | 0 | 0 | 0 |
| Sodium Fluoride | 0 | 0 | 0 |
| Full Key Mint Booster | 0.5 | 0.5 | 0.5 |
| Peppermint Flavor | 0 | 0 | 0 |
| Cooling Flavor WS-5 | 0.45 | 0.45 | 0.45 |
| Blue No.1 | 0.025 | 0.025 | 0.025 |
| Powder Flavor | 4 | 4 | 4 |
| CPC | 1.6 | 0 | 0 |
| | 100 | 100 | 100 |
| Micro Result: | Pass | Fail | Pass |

| | | | |
|---|---|---|---|
| * not claimed | | | |

### EXAMPLE 3

The level of directly compressible sugar alcohols affects disintegration of tablets. The experiments outlined in Table 6 below test a formula with 11 wt. % mannitol and 8 wt. % sorbitol, a formula without sorbitol, and a formula without powder flavor which contains acacia gum as a carrier. The formula with all mannitol and no sorbitol showed a faster disintegration time. Removal of the powder flavor did not improve disintegration time.

**Table 6**

| Ingredient | **Formulation 13 (wt. %)** * | **Formulation 14 (wt. %)** * | **Formulation 15 (wt. %) *** |
|---|---|---|---|
| Sodium Carbonate | 24 | 24 | 24 |
| Citric Acid | 24 | 24 | 24 |
| Poloxamer 407 | 1 | 1 | 1 |
| Mannitol | 11 | 19 | 11 |
| Potassium Sorbate | 6 | 6 | 6 |
| Sorbitol | 8 | 0 | 8 |
| Sucralose | 1 | 1 | 1 |
| Flavorant | 4 | 4 | 0 |
| | | | |
| Disintegration time at room temperature (min.) | 4.49 | 4:00 | >6:00 |

| | | | |
|---|---|---|---|
| * not claimed | | | |

The high amounts of compressible material led to disintegration problems. For example, initial tests showed that tablets containing 8 wt. % sorbitol and 11 wt. % mannitol did not fully dissolve. The sodium bicarbonate and citric acid present in the tablet completed an effervescent reaction but a skeleton of the directly compressible material (i.e., mannitol and sorbitol) remained intact by attractive forces that are too strong to be separated by effervescent action. The formula containing mannitol and no sorbitol (i.e., Formulation 14) showed a faster disintegration time than Formulation 13. Removal of the flavorant in Formulation 15 did not improve disintegration time. Subsequent formulas have sorbitol removed.

Subsequent compositions were prepared in which the level of mannitol was reduced to 11% and sorbitol was removed, as summarized in Table 7 below.

**Table 7**

| Ingredient | **Formulation 16 (wt. %) *** | **Formulation 17 (wt. %) *** | **Formulation 18 (wt. %) *** | **Formulation 19 (wt. %) *** | **Formulation 20 (wt. %)*** |
|---|---|---|---|---|---|
| Sucralose | 1 | 1 | 1 | 1 | 1 |
| Sodium Bicarbonate | 24 | 24 | 25 | 28 | 27 |
| Citric Acid | 24 | 26 | 26 | 29 | 29 |
| Poloxamer 407 | 1 | 1 | 0 | 0 | 0.2 |
| Mannitol | 16 | 17 | 17 | 11 | 11 |
| Sorbitol | 3 | 0 | 0 | 0 | 0 |
| Sodium Benzoate | 20 | 20 | 20 | 20 | 20 |
| Potassium Sorbate | 6 | 6 | 6 | 6 | 6 |
| Flavorants and colorants | 5 | 5 | 5 | 5 | 5 |
| Total | 100 | 100 | 100 | 100 | 100 |
| | | | | | |
| Disintegration time at room temperature (min.) | >10 | >10 | >10 | 3:40 | 5:54 |
| pH | 5.4 | 5.4 | 5.3 | 5.2 | 5.2 |

| | | | | | |
|---|---|---|---|---|---|
| * not claimed | | | | | |

The removal of sorbitol resulted in significantly reduced occurrence of directly compressible material skeleton. Poloxamer was completely removed from Formulations 18 and 19. Formulation 19, containing a reduced amount of directly compressible material (i.e., mannitol and sorbitol), resulted in the fastest disintegration time; however, the solution contained aesthetically unpleasing particles suspended eventually dissolving over several additional minutes. Formulation 20 contained poloxamer at a lower level of 0.2 wt. %. This solved the precipitate issue, and also resulted in an acceptable dissolution time.

### EXAMPLE 4

Effervescent tablets are hygroscopic and are prone to reacting with humidity in the air. Premature reaction of sodium bicarbonate with citric acid leads to many issues including change in the appearance of tablet. It is important to package the tablets in a container that offers good moisture barrier and has proper closure. The following experiments explored various packaging materials to find the optimal packaging material for this tablet.

Tablets according to the present disclosure were aged for 3 months in a glass jar with a child-resistant cap containing a cotton ball and a desiccant. The tablets changed in texture and color, and some bloating was observed.

Tablets according to the present disclosure were further tested in CSP APTAR packaging and compared to glass with desiccant combination. The CSP packaging passed in terms of color, odor and appearance. The tablets were smooth in texture and color did not change after 3 months under accelerated aging conditions (i.e., at 40°C) as compared to the glass packaging.

### EXAMPLE 5

The following are representative and non-claimed, comparative tablets (e.g., solid oral care composition) of the present disclosure:

**Table 8 - Representative Formulation (Tablet)**

| **Material** | **Composition 1 (wt. %) *** | **Composition 2 (wt. %) *** | **Composition 3 (wt. %)** |
|---|---|---|---|
| Poloxamer 407 | 1 | 1 | 0.2 |
| Polyvinylpyrrolidone (PVP) | 4.5 | 4.5 | 0 |
| PVP-vinyl acetate copolymer | 0 | 0 | 4.2 |
| Mannitol | 8 | 8 | 10 |
| Sodium benzoate | 20 | 20 | 15 |
| Potassium sorbate | 2 | 2 | 6 |
| Sodium bicarbonate | 24 | 24 | 18 |
| Citric acid | 28 | 28 | 25 |
| Sorbitol | 7 | 7 | 10.6 |
| Sodium fluoride | 2 | 0.8 | 0 |
| Arabic gum | 2 | 2 | 0 |
| Sucralose | 0 | 0 | 0.8 |
| Sodium Lauryl Sulfate | 0 | 0 | 2.5 |
| Hydroxypropylcellulose | 0 | 0 | 2 |
| Magnesium Stearate | 0 | 0 | 0.3 |
| Flavorants and colorants | 1-2 | 1-2 | 5.4 |
| Total | 100 | 100 | 100 |

| | | | |
|---|---|---|---|
| * not claimed | | | |

### EXAMPLE 6

Tablets were prepared using the lubrication systems shown in Table 9 below. These compositions are for comparative purposes.

**Table 9**

| | **Lubricant System** | **Effect** |
|---|---|---|
| 1 | 2% SLS (sodium lauryl sulfate), 0.2% Magnesium Stearate | Sufficient lubrication, Smooth tablets |
| 2 | 2% SLS, 0.2% Sodium Stearyl Fumarate | Edges rough some pitting and picking |
| 3 | 2% SLS, 0.2% Poloxamer | Deep pitting and picking |
| 4 | 2% Poloxamer, 0.2% Magnesium Stearate | Sufficient lubrication, Smooth tablets |
| 5 | 2% SLS, 0.2% Magnesium Stearate, 0.2% Sodium Stearyl Fumarate | Sufficient lubrication, Smooth tablets |
| 6 | 3% SLS | Pitting and picking observed |
| 7 | 2% Poloxamer 0.2% Magnesium Stearate, 0.2% Sodium Stearyl Fumarate | Pitting and picking observed |

The best results were obtained with Systems 1 and 4. Based on these results, a lubricant system including SLS, Magnesium Stearate and Poloxamer 407 is expected to provide a smooth tablet and optimal handling characteristics.

## Claims

1. A solid oral care composition comprising:
a sugar alcohol;
a lubricant system comprising:
a poloxamer being present in an amount sufficient to maintain stability of the composition for a period of 3-30 days when dissolved in water wherein the amount of the poloxamer is from 0.1 to 0.5 wt.%, relative to the total weight of the solid oral care composition,
an anionic surfactant, and
a stearate; and
a preservative comprising sodium benzoate and potassium sorbate, wherein the sodium benzoate is present an amount of from 15 to 25 wt.%, relative to the total weight of the composition, and the potassium sorbate is present in an amount of 1 to 8% by weight relative to the total weight of the solid oral care composition.

2. The solid oral care composition according to claim 1, wherein:
the anionic surfactant, preferably sodium lauryl sulfate, is present in an amount from 1 to 5%, by weight relative to the total weight of the solid oral care composition; and
the stearate, preferably magnesium stearate, is present in an amount from 0.1 to 0.5%, by weight relative to the total weight of the solid oral care composition.

3. The solid oral care composition according to any of the preceding claims, further comprising PVP or a copolymer thereof; wherein the PVP or copolymer thereof and the poloxamer are present in a weight ratio of the PVP or a copolymer thereof to the poloxamer of 9:1 to 1:1, wherein the weight is relative to the total weight of the solid oral care composition.

4. The solid oral care composition according to any of the preceding claims, wherein the sugar alcohol is selected from the group consisting of mannitol, sorbitol, erythritol, xylitol, lactitol, maltitol, isomalt, and combinations thereof, preferably wherein the sugar alcohol is mannitol and sorbitol in a weight ratio of mannitol to sorbitol of 0.8:1 to 1.2:1.

5. The solid oral care composition according to any of the preceding claims, wherein the solid oral care composition is in the form of a tablet, powder, or granule.

6. The solid oral care composition according to any of the preceding claims, further comprising a polymer binder selected from starches, natural gums, cellulose gums, microcrystalline cellulose, maltodextrins, methylcellulose, cellulose ethers, sodium carboxymethylcellulose, ethylcellulose, gelatin, polyethylene glycol, cross-linked polyvinylpyrrolidone, pectins, alginates, polyacrylamides, polyvinyloxazolidone, polyvinyl alcohols, and mixtures thereof, wherein the polymer binder is present in an amount of 1 to 5% by weight relative to the total weight of the solid oral care composition.

7. The solid oral care composition according to any of the preceding claims, wherein the solid oral care composition is a tablet that comprises:
a) poloxamer 407;
b) 1 to 5% of an anionic surfactant, by weight relative to the total weight of the solid oral care composition;
c) 0.1 to 0.5% of a stearate, by weight relative to the total weight of the solid oral care composition;
d) 18 to 22% of a sugar alcohol, relative to the total weight of the solid oral care composition; and
e) up to 25% of a total amount of a preservative by weight relative to the total weight of the solid oral care composition, the preservative comprising 20% of sodium benzoate and 2% of potassium sorbate by weight relative to the total weight of the solid oral care composition; and
f) 2 to 6% of PVP or a copolymer thereof, relative to the total weight of the solid oral care composition.

8. The composition according to claim 7, further comprising:
g) 1 to 5% of a binder, by weight relative to the total weight of the solid oral care composition;
h) 20 to 30% of a buffering agent, by weight relative to the total weight of the solid oral care composition; and
i) 15 to 20% of a carbonate base, by weight relative to the total weight of the solid oral care composition.

9. A solid oral care composition according to any one of claims 1-8 for use in a method of controlling a bacterial population in the oral cavity, the method comprising dissolving the solid oral care composition in water to form an aqueous solution, and storing the aqueous solution in a container for at least 3 days, wherein the solid oral care composition is a tablet, and wherein the method further comprises administration of said oral care composition to the oral cavity.

10. The solid oral care composition for use according to claim 9, wherein the solid oral care composition is dissolved in 40mL water per gram of the solid oral care composition; and further comprising the step of using the aqueous solution daily for a period of at least 3 days.

11. A kit comprising (i) the solid oral care composition according to any one of claims 1-8; and (ii) a container for holding a liquid for a prolonged period of time.

## Patentansprüche

1. Feste Mundpflegezusammensetzung, umfassend:
einen Zuckeralkohol;
ein Gleitmittelsystem, umfassend:
ein Poloxamer, das in einer Menge vorhanden ist, die ausreicht, um die Stabilität der Zusammensetzung für einen Zeitraum von 3 bis 30 Tagen aufrechtzuerhalten, wenn es in Wasser gelöst ist, wobei die Menge des Poloxamers 0,1 bis 0,5 Gew.-% beträgt, bezogen auf das Gesamtgewicht der festen Mundpflegezusammensetzung,
ein anionisches Tensid und
ein Stearat; und
ein Konservierungsmittel, das Natriumbenzoat und Kaliumsorbat umfasst, wobei das Natriumbenzoat in einer Menge von 15 bis 25 Gew.-%, bezogen auf das Gesamtgewicht der Zusammensetzung, und das Kaliumsorbat in einer Menge von 1 bis 8 Gew.-%, bezogen auf das Gesamtgewicht der festen Mundpflegezusammensetzung, vorhanden ist.

2. Feste Mundpflegezusammensetzung gemäß Anspruch 1, wobei:
das anionische Tensid, vorzugsweise Natriumlaurylsulfat, in einer Menge von 1 bis 5 Gew.-%, bezogen auf das Gesamtgewicht der festen Mundpflegezusammensetzung, vorhanden ist und
das Stearat, vorzugsweise Magnesiumstearat, in einer Menge von 0,1 bis 0,5 Gew.-%, bezogen auf das Gesamtgewicht der festen Mundpflegezusammensetzung, vorhanden ist.

3. Feste Mundpflegezusammensetzung gemäß einem der vorstehenden Ansprüche, die ferner PVP oder ein Copolymer davon umfasst; wobei das PVP oder Copolymer davon und das Poloxamer in einem Gewichtsverhältnis von PVP oder Copolymer davon zu Poloxamer von 9:1 bis 1:1 vorliegen, wobei das Gewicht auf das Gesamtgewicht der festen Mundpflegezusammensetzung bezogen ist.

4. Feste Mundpflegezusammensetzung gemäß einem der vorstehenden Ansprüche, wobei der Zuckeralkohol aus der Gruppe ausgewählt ist, die aus Mannitol, Sorbit, Erythrit, Xylit, Lactit, Maltit, Isomalt und Kombinationen davon besteht, vorzugsweise , wobei der Zuckeralkohol Mannitol und Sorbit in einem Gewichtsverhältnis von Mannitol zu Sorbit von 0,8:1 bis 1,2:1 ist.

5. Feste Mundpflegezusammensetzung gemäß einem der vorstehenden Ansprüche, wobei die feste Mundpflegezusammensetzung in Form einer Tablette, eines Pulvers oder eines Granulats vorliegt.

6. Feste Mundpflegezusammensetzung gemäß einem der vorstehenden Ansprüche, die ferner ein Polymerbindemittel umfasst, ausgewählt aus Stärken, natürlichen Gummiarten, Cellulosegummiarten, mikrokristalliner Cellulose, Maltodextrinen, Methylcellulose, Celluloseethern, Natriumcarboxymethylcellulose, Ethylcellulose, Gelatine, Polyethylenglykol, vernetztem Polyvinylpyrrolidon, Pektinen, Alginaten, Polyacrylamiden, Polyvinyloxazolidon, Polyvinylalkoholen und Mischungen davon, wobei das Polymerbindemittel in einer Menge von 1 bis 5 Gew.-%, bezogen auf das Gesamtgewicht der festen Mundpflegemittelzusammensetzung, vorhanden ist.

7. Feste Mundpflegezusammensetzung gemäß einem der vorstehenden Ansprüche, wobei die feste Mundpflegezusammensetzung eine Tablette ist, die umfasst:
a) Poloxamer 407;
b) 1 bis 5 Gew.-% eines anionischen Tensids, bezogen auf das Gesamtgewicht der festen Mundpflegezusammensetzung;
c) 0,1 bis 0,5 Gew.-% eines Stearats, bezogen auf das Gesamtgewicht der festen Mundpflegezusammensetzung;
d) 18 bis 22 Gew.-% eines Zuckeralkohols, bezogen auf das Gesamtgewicht der festen Mundpflegezusammensetzung; und
e) bis zu 25 Gew.-% einer Gesamtmenge eines Konservierungsmittels, bezogen auf das Gesamtgewicht der festen Mundpflegezusammensetzung, wobei das Konservierungsmittel Folgendes umfasst 20 Gew.-% Natriumbenzoat und 2 Gew.-% Kaliumsorbat e, bezogen auf das Gesamtgewicht der festen Mundpflegezusammensetzung; und
f) 2 bis 6 % PVP oder eines Copolymers davon, bezogen auf das Gesamtgewicht der festen Mundpflegezusammensetzung.

8. Zusammensetzung gemäß Anspruch 7, die ferner umfasst:
g) 1 bis 5 Gew.-% eines Bindemittels, bezogen auf das Gesamtgewicht der festen Mundpflegezusammensetzung;
h) 20 bis 30 Gew.-% eines Puffermittels, bezogen auf das Gesamtgewicht der festen Mundpflegezusammensetzung; und
i) 15 bis 20 Gew.-% einer Karbonatbasis, bezogen auf das Gesamtgewicht der festen Mundpflegezusammensetzung.

9. Feste Mundpflegezusammensetzung gemäß einem der Ansprüche 1 bis 8 zur Verwendung in einem Verfahren zur Kontrolle einer Bakterienpopulation in der Mundhöhle, wobei das Verfahren das Auflösen der festen Mundpflegezusammensetzung in Wasser unter Bildung einer wässrigen Lösung und das Lagern der wässrigen Lösung in einem Behälter für mindestens 3 Tage umfasst, wobei die feste Mundpflegezusammensetzung eine Tablette ist, und wobei das Verfahren ferner die Verabreichung der Mundpflegezusammensetzung an die Mundhöhle umfasst, .

10. Feste Mundpflegezusammensetzung zur Verwendung gemäß Anspruch 9, wobei die feste Mundpflegezusammensetzung in 40 ml Wasser pro Gramm der festen Mundpflegezusammensetzung gelöst wird und ferner umfassend den Schritt der täglichen Verwendung der wässrigen Lösung über einen Zeitraum von mindestens 3 Tagen.

11. Kit, umfassend (i) die feste Mundpflegezusammensetzung gemäß einem der Ansprüche 1 bis 8 und (ii) einen Behälter zum Aufbewahren einer Flüssigkeit über einen längeren Zeitraum.

## Revendications

1. Composition solide pour soins bucco-dentaires, comprenant :
un alcool de sucre ;
un système lubrifiant comprenant :
un poloxamère étant présent en une quantité suffisante pour maintenir la stabilité de la composition pendant une période de 3 à 30 jours lorsqu'il est dissous dans de l'eau, dans lequel la quantité de poloxamère est de 0,1 à 0,5 % en poids, par rapport au poids total de la composition solide pour soins bucco-dentaires,
un tensioactif anionique ; et
un stéarate ; et
un conservateur comprenant du benzoate de sodium et du sorbate de potassium, dans lequel le benzoate de sodium est présent en une quantité de 15 à 25 % en poids, par rapport au poids total de la composition, et le sorbate de potassium est présent en une quantité de 1 et 8 % en poids par rapport au poids total de la composition solide pour soins bucco-dentaires.

2. Composition solide pour soins bucco-dentaires selon la revendication 1, dans laquelle :
le tensioactif anionique, de préférence du laurylsulfate de sodium, est présent en une quantité de 1 à 5 %, en poids par rapport au poids total de la composition solide pour soins bucco-dentaires ; et
le stéarate, de préférence du stéarate de magnésium, est présent en une quantité de 0,1 à 0,5 % en poids, par rapport au poids total de la composition solide pour soins bucco-dentaires.

3. Composition solide pour soins bucco-dentaires selon l'une quelconque des revendications précédentes, comprenant en outre de la PVP ou un copolymère de celle-ci ; dans laquelle la PVP ou le copolymère de celle-ci et le poloxamère sont présents en un rapport pondéral de la PVP ou d'un copolymère de celle-ci au poloxamère de 9:1 à 1:1, dans laquelle le poids est exprimé par rapport au poids total de la composition solide pour soins bucco-dentaires.

4. Composition solide pour soins bucco-dentaires selon l'une quelconque des revendications précédentes, dans laquelle l'alcool de sucre est choisi dans le groupe constitué par le mannitol, le sorbitol, l'érythritol, le xylitol, le lactitol, le maltitol, l'isomalt et les combinaisons de ceux-ci, de préférence dans laquelle l'alcool de sucre est le mannitol et le sorbitol en un rapport pondéral du mannitol au sorbitol de 0,8:1 à 1,2:1.

5. Composition solide pour soins bucco-dentaires selon l'une quelconque des revendications précédentes, dans laquelle la composition solide pour soins bucco-dentaires est sous la forme d'un comprimé, d'une poudre ou d'un granulé.

6. Composition solide pour soins bucco-dentaires selon l'une quelconque des revendications précédentes, comprenant en outre un liant polymère choisi parmi les amidons, les gommes naturelles, les gommes de cellulose, la cellulose microcristalline, les maltodextrines, la méthylcellulose, les éthers de cellulose, la carboxyméthyl-cellulose sodique, l'éthylcellulose, la gélatine, le polyéthylène glycol, la polyvinylpyrrolidone réticulée, les pectines, les alginates, les polyacrylamides, la polyvinyloxazolidone, les alcools polyvinyliques et les mélanges de ceux-ci, dans laquelle le liant polymère est présent en une quantité de 1 à 5 % en poids par rapport au poids total de la composition solide pour soins bucco-dentaires.

7. Composition solide pour soins bucco-dentaires selon l'une quelconque des revendications précédentes, dans laquelle la composition solide pour soins bucco-dentaires est un comprimé qui comprend :
a) du poloxamère 407 ;
b) 1 à 5 % d'un tensioactif anionique, en poids par rapport au poids total de la composition solide pour soins bucco-dentaires ;
c) 0,1 à 0,5 % d'un stéarate, en poids par rapport au poids total de la composition solide pour soins bucco-dentaires ;
d) 18 à 22 % d'un alcool de sucre, par rapport au poids total de la composition solide pour soins bucco-dentaires ; et
e) jusqu'à 25 % d'une quantité totale d'un conservateur, en poids par rapport au poids total de la composition solide pour soins bucco-dentaires, le conservateur comprenant 20 % de benzoate de sodium et 2 % de sorbate de potassium, en poids par rapport au poids total de la composition solide pour soins bucco-dentaires ; et
f) 2 à 6 % de PVP ou d'un copolymère de celle-ci, par rapport au poids total de la composition solide pour soins bucco-dentaires.

8. Composition selon la revendication 7, comprenant en outre :
g) 1 à 5 % d'un liant, en poids par rapport au poids total de la composition solide pour soins bucco-dentaires ;
h) 20 à 30 % d'un agent tampon, en poids par rapport au poids total de la composition solide pour soins bucco-dentaires ; et
i) 15 à 20 % d'une base de carbonate, en poids par rapport au poids total de la composition solide pour soins bucco-dentaires.

9. Composition solide pour soins bucco-dentaires selon l'une quelconque des revendications 1 à 8, pour une utilisation dans un procédé de lutte contre une population bactérienne dans la cavité buccale, le procédé comprenant la dissolution de la composition solide pour soins bucco-dentaires dans l'eau pour former une solution aqueuse, et le stockage de la solution aqueuse dans un récipient pendant au moins 3 jours, dans laquelle la composition solide pour soins bucco-dentaires est un comprimé , et dans laquelle le procédé comprend en outre l'administration de ladite composition solide pour soins bucco-dentaires à la cavité buccale.

10. Composition solide pour soins bucco-dentaires pour une utilisation selon la revendication 9, dans laquelle la composition solide pour soins bucco-dentaires est dissoute dans 40 ml d'eau par gramme de la composition solide pour soins bucco-dentaires ; et comprenant en outre l'étape d'utilisation quotidienne de la solution aqueuse pendant une période d'au moins 3 jours.

11. Kit comprenant (i) la composition solide pour soins bucco-dentaires selon l'une quelconque des revendications 1 à 8 ; et (ii) un récipient pour contenir un liquide pendant une période de temps prolongée.
